# EUROPEAN PATENT APPLICATION

(11) **EP 4 800 101 A1**
(43) Date of publication of application: **02.09.2026**
(21) Application number: 24882370.0
(22) Date of filing: 22.10.2024
(51) Int. Cl.: C12N 7/01, A61K 39/145, A61K 39/225, A61K 39/245, A61P 31/14, A61P 31/16, A61P 31/22, C12N 15/38, C12N 15/44, C12N 15/50

(54) **RECOMBINANT LIVE ATTENUATED HERPES SIMPLEX VIRUS TYPE 1 VACCINE**

(30) Priority: 23.10.2023 JP 2023182027
(71) Applicant: The University of Tokyo, Bunkyo-ku, Tokyo 113-8654 (JP); KM Biologics Co., Ltd., Kumamoto-shi, Kumamoto 860-8568 (JP)
(72) Inventor: KAWAGUCHI, Yasushi, Tokyo 113-8654 (JP); KOYANAGI, Naoto, Tokyo 113-8654 (JP); SUGIURA, Takatoshi, Kikuchi-shi, Kumamoto 869-1298 (JP)
(74) Representative: Graf von Stosch Patentanwaltsgesellschaft mbH
(86) International application number: PCT/JP2024/037505
(87) International publication number: WO 2025/089259

(57) **Abstract**

A virus of the present disclosure is a multiple mutant virus in which: two or more genes of herpes simplex virus type 1 (HSV-1) are modified; and a modification of the two or more genes involves a loss or reduction in gene function.

## Description

### Technical Field

The present invention relates to recombinant herpes simplex virus type 1 and a recombinant live attenuated herpes simplex virus type 1 vaccine.

### Background Art

Human herpes simplex virus (HSV) is a pathogen that is widely spread in humans. HSV that is a dsDNA virus belongs to Alphaherpesvirinae and has two serotypes HSV-1 and HSV-2. HSV causes various diseases such as encephalitis, meningitis, herpes labialis, genital herpes, skin diseases, corneal herpes, and neonatal systemic herpes in humans. Thus, HSV is a virus of critical medical health importance, and an antiviral agent such as acyclovir or valacyclovir actually has been developed.

However, anti-HSV agents that have been developed to date inhibit replication of viral DNA, and thus an effect thereof on HSV in a DNA state with which the interior of the ganglion is latently infected is limited. In a case where a timing of administration of an anti-HSV agent is missed due to, for example, cessation of medication, medication reduction, or forgetting to take medicine, a therapeutic effect of the anti-HSV agent may be reduced. This prevents achievement of fundamental disease control. In order to overcome such a situation, it is necessary to develop a vaccine that is effective in preventing primary infection and recurrence and that is highly safe.

A pathogen causing an infectious disease is roughly divided into a class I pathogen that makes it possible to obtain a sufficient effect with an existing vaccine and a class II pathogen that makes it impossible to acquire sufficient protective immunity with an existing vaccine or pathogen infection history. An ingenious immune evasion mechanism possessed by class II pathogens is pointed out as a reason why it is difficult to defend against the class II pathogens. HSV is classified as a class II pathogen. This is considered to be because HSV has an immune evasion mechanism and ingeniously evades a host immune response. Regarding HSV vaccine development, a study mainly on subunit vaccines has been attempted until now, and the subunit vaccines are all insufficiently effective (Non-patent Literatures 1 to 3).

It is pointed out that induction of CD4-positive and CD8-positive T cells is important for pathological control, and induction of tissue-resident memory T cells is also important (Non-patent Literatures 4 to 7). It is also known that a live attenuated vaccine can strongly induce humoral immunity and cellular immunity (Non-patent Literatures 8 to 11).

A gene Us3 of HSV-1 encodes protein kinase Us3 and plays an important role in pathogenicity expression. A Us3 mutant is shown to have reduced pathogenicity (Non-patent Literatures 12 to 16 of a deletion variant) and is shown not to be essential for viral propagation in cultured cells (Non-patent Literatures 17 to 19 of a deletion variant). Furthermore, Us3 of HSV-1 is shown to evade cytotoxic T cells (CTLs) by suppressing cell surface expression of major histocompatibility antigen class I (MHC-I) (Non-patent Literature 20).

A gene UL13 of HSV-1 encodes protein kinase UL13 and plays an important role in pathogenicity expression. A UL13 mutant is shown to have reduced pathogenicity (Non-patent Literatures 21 and 22 of a deletion variant) and is shown not to be essential for viral propagation in cultured cells (Non-patent Literatures 17 and 22 of a deletion variant). Furthermore, UL13 of HSV-1 is shown to contribute to efficient viral propagation by suppressing expression of chemokine ligand 9 (CXCL9) and inhibiting invasion of CTLs into an infection site (Non-patent Literature 22).

### Citation List

### [Non-patent Literature]

[Non-patent Literature 1]
   Kim, H.C. et al., Vaccines against Genital Herpes: Where Are We? Vaccines (Basel) 2020 8: 420
[Non-patent Literature 2]
   Egan, K. et al., Vaccines to prevent genital herpes. Transl Res 2020 220: 138-152
[Non-patent Literature 3]
   Truong, N.R. et al., Mechanisms of Immune Control of Mucosal HSV Infection: A Guide to Rational Vaccine Design. Front Immunol 2019 10: 373
[Non-patent Literature 4]
   Chentoufi, A.A. et al., Towards a rational design of an asymptomatic clinical herpes vaccine: the old, the new, and the unknown. Clin Dev Immunol 2012 2012: 187585
[Non-patent Literature 5]
   Zhu, J. et al., Persistence of HIV-1 receptor-positive cells after HSV-2 reactivation is a potential mechanism for increased HIV-1 acquisition. Nat Med 2009 15: 886-92
[Non-patent Literature 6]
   Zhu, J. et al., Virus-specific CD8+ T cells accumulate near sensory nerve endings in genital skin during subclinical HSV-2 reactivation. J Exp Med 2007 204: 595-603
[Non-patent Literature 7]
   Posavad, C.M. et al., Enrichment of herpes simplex virus type 2 (HSV-2) reactive mucosal T cells in the human female genital tract. Mucosal Immunol 2017 10: 1259-69
[Non-patent Literature 8]
   Lauring, A.S. et al., Rationalizing the development of live attenuated virus vaccines. Nat Biotechnol 2010 28: 573-9
[Non-patent Literature 9]
   Jang, Y.H. et al., Principles underlying rational design of live attenuated influenza vaccines. Clin Exp Vaccine Res 2012 1: 35-49
[Non-patent Literature 10]
   Cote-Gravel, J. et al., Vaccination with a live-attenuated small-colony variant improves the humoral and cell-mediated responses against Staphy-lococcus aureus. PLoS One 2019 14: e0227109
[Non-patent Literature 11]
   Zhu, J. et al., Herpes Simplex Vaccines: Prospects of Live-attenuated HSV Vaccines to Combat Genital and Ocular infections. Curr Clin Microbiol Rep 2015 2: 125-36
[Non-patent Literature 12]
   Koyanagi, N. et al., Role of herpes simplex virus 1 Us3 in viral neuroinvasiveness. Microbiol Immunol 2014 58: 31-7
[Non-patent Literature 13]
   Morimoto, T. et al., Differences in the regulatory and functional effects of the Us3 protein kinase activities of herpes simplex virus 1 and 2. J Virol 2009 83: 11624-34
[Non-patent Literature 14]
   Imai, T. et al., Effects of phosphorylation of herpes simplex virus 1 envelope glycoprotein B by Us3 kinase in vivo and in vitro. J Virol 2010 84: 153-62
[Non-patent Literature 15]
   Sagou, K. et al., Regulation of the catalytic activity of herpes simplex virus 1 protein kinase Us3 by autophosphorylation and its role in pathogenesis. J Virol 2009 83: 5773-83
[Non-patent Literature 16]
   Kato, A. et al., Phosphorylation of a herpes simplex virus 1 dUTPase by a viral protein kinase, Us3, dictates viral pathogenicity in the central nervous system but not at the periphery. J Virol 2014 88: 2775-85
[Non-patent Literature 17]
   Gershburg, S. et al., The UL13 and US3 Protein Kinases of Herpes Simplex Virus 1 Cooperate to Promote the Assembly and Release of Mature, Infectious Virions. PLos One 2015 10: e0131420
[Non-patent Literature 18]
   Kato, A. et al., Identification of a physiological phosphorylation site of the herpes simplex virus 1-encoded protein kinase Us3 which regulates its optimal catalytic activity in vitro and influences its function in infected cells. J Virol 2008 82: 6172-89
[Non-patent Literature 19]
   Shindo, K. et al., Characterization of a Herpes Simplex Virus 1 (HSV-1) Chimera in Which the Us3 Protein Kinase Gene Is Replaced with the HSV-2 Us3 Gene. J Virol 2015 90: 457-73
[Non-patent Literature 20]
   Imai, T. et al., Us3 kinase encoded by herpes simplex virus 1 mediates downregulation of cell surface major histocompatibility complex class I and evasion of CD8+ T cells. PLoS One 2013 8: e72050
[Non-patent Literature 21]
   Shibaki, T. et al., Participation of type I interferon in the decreased virulence of the UL13 gene-deleted mutant of herpes simplex virus type 1. J Interferon Cytokine Res 2001 21: 279-85
[Non-patent Literature 22]
   Koyanagi, N. et al., Herpes simplex virus-1 evasion of CD8+ T cell accumulation contributes to viral encephalitis. J Clin Invest 2017 127: 3784-95
[Non-patent Literature 23]
   Gordon, YJ. et al., HSV-1 thymidine kinase negative vaccine: pathogenicity, protection, and perils. Curr Eye Res 1987 6: 151-9
[Non-patent Literature 24]
   Coen, DM. et al., Thymidine kinase-negative herpes simplex virus mutants establish latency in mouse trigeminal ganglia but do not reactivate. Proc Natl Acad Sci U S A 1989 86: 4736-40
[Non-patent Literature 25]
   Koshizuka, T. et al., Protection from lethal herpes simplex virus type 1 infection by vaccination with a UL41-deficient recombinant strain. Fukushima J Med Sci 2016 62: 36-42
[Non-patent Literature 26]
   Shahnazaryan, D. et al., Herpes simplex virus 1 targets IRF7 via ICPO to limit type I IFN induction. Sci Rep 2020 10: 22216
[Non-patent Literature 27]
   Zhang, L. et al., HSV-1-encoded ICPO degrades the host deubiquitinase BRCC36 to antagonize interferon antiviral response. Mol Immunol 2021 135: 28-35
[Non-patent Literature 28]
   McMenamin, MM. et al., A gamma34.5 mutant of herpes simplex 1 causes severe inflammation in the brain. Neuroscience 1998 83: 1225-37
[Non-patent Literature 29]
   Gobeil, PA. et al., Herpes simplex virus γ34.5 interferes with autophagosome maturation and antigen presentation in dendritic cells. mBio 2012 16: e00267-12
[Non-patent Literature 30]
   Pyles, RB. et al., Evidence that the herpes simplex virus type 1 uracil DNA glycosylase is required for efficient viral replication and latency in the murine nervous system. J Virol 1994 68: 4963-72
[Non-patent Literature 31]
   Jun, PY. et al., The UL4 gene of herpes simplex virus type 1 is dispensable for latency, reactivation and pathogenesis in mice. J Gen Virol 1998 79: 1603-11
[Non-patent Literature 32]
   Fujii, H. et al., Role of the nuclease activities encoded by herpes simplex virus 1 UL12 in viral replication and neurovirulence. J Virol 2014 88: 2359-64
[Non-patent Literature 33]
   Jacobson, JG. et al., Importance of the herpes simplex virus UL24 gene for productive ganglionic infection in mice. Virology 1998 242: 161-9
[Non-patent Literature 34]
   Imai, T. et al., Role of the herpes simplex virus 1 Us3 kinase phosphorylation site and endocytosis motifs in the intracellular transport and neurovirulence of envelope glycoprotein B. J Virol 2011 85: 5003-15
[Non-patent Literature 35]
   Kobayashi, R. et al., Function of the Herpes Simplex Virus 1 Small Capsid Protein VP26 Is Regulated by Phosphorylation at a Specific Site. J Virol 2015 89: 6141-7
[Non-patent Literature 36]
   Samady, L. et al., Deletion of the virion host shutoff protein (vhs) from herpes simplex virus (HSV) relieves the viral block to dendritic cell activation: potential of vhs- HSV vectors for dendritic cell-mediated immunotherapy. J Virol 2003 77: 3768-76
[Non-patent Literature 37]
   Paludan, SR. et al., Recognition of herpesviruses by the innate immune system. Nat Rev Immunol 2011 11: 143-54
[Non-patent Literature 38]
   Everly, DN Jr. et al., mRNA degradation by the virion host shutoff (Vhs) protein of herpes simplex virus: genetic and biochemical evidence that Vhs is a nuclease. J Virol 2002 76: 8560-71
[Non-patent Literature 39]
   Friedman, HM. et al., Immune evasion properties of herpes simplex virus type 1 glycoprotein gC. J Virol 1996 70: 4253-60
[Non-patent Literature 40]
   Lubinski, JM. et al., Herpes simplex virus type 1 glycoprotein gC mediates immune evasion in vivo. J Virol 1998 72: 8257-63
[Non-patent Literature 41]
   Visalli, RJ. et al., Mutation of the herpes simplex virus 1 KOS UL45 gene reveals dose dependent effects on central nervous system growth. Arch Virol 2002 147: 519-32
[Non-patent Literature 42]
   Deschamps, T. et al., Evasion of the STING DNA-Sensing Pathway by VP11/12 of Herpes Simplex Virus 1. J Virol 2017 91: e00535-17
[Non-patent Literature 43]
   You, H. et al., Herpes Simplex Virus 1 Tegument Protein UL46 Inhibits TANK-Binding Kinase 1-Mediated Signaling. mBio 2019 10: e00919-19
[Non-patent Literature 44]
   Ariza, ME. et al., Human herpesviruses-encoded dUTPases: a family of proteins that modulate dendritic cell function and innate immunity. Front Microbiol 2014 5: 504
[Non-patent Literature 45]
   Kato, A. et al., Identification of a herpes simplex virus 1 gene encoding neurovirulence factor by chemical proteomics. Nat Commun 2020 11: 4894
[Non-patent Literature 46]
   Berkowitz, C. et al., Herpes simplex virus type 1 (HSV-1) UL56 gene is involved in viral intraperitoneal pathogenicity to immunocompetent mice. Arch Virol 1994 134: 73-83
[Non-patent Literature 47]
   Zheng, ZQ. et al., Herpes simplex virus protein UL56 inhibits cGAS-Mediated DNA sensing to evade antiviral immunity. Cell Insight 2022 1: 100014
[Non-patent Literature 48]
   Lu, X. et al., The Us2 Gene Product of Herpes Simplex Virus 2 modulates NF-κB activation by targeting TAK1. Sci Rep 2017 7: 8396.
[Non-patent Literature 49]
   Cabrera, JR. et al., Secreted herpes simplex virus-2 glycoprotein G modifies NGF-TrkA signaling to attract free nerve endings to the site of infection. PLoS Pathog 2015 11: e1004571.
[Non-patent Literature 50]
   Martinez-Martin, N. et al., Herpes simplex virus particles interact with chemokines and enhance cell migration. J Gen Virol 2016 97: 3007-16
[Non-patent Literature 51]
   Zhou, G. et al., Glycoprotein D or J delivered in trans blocks apoptosis in SK-N-SH cells induced by a herpes simplex virus 1 mutant lacking intact genes expressing both glycoproteins. J Virol 2000 74: 11782-91
[Non-patent Literature 52]
   Jerome, KR. et al., HSV and glycoprotein J inhibit caspase activation and apoptosis induced by granzyme B or Fas. J Immunol 2001 167: 3928-35
[Non-patent Literature 53]
   Kato, A. et al., Roles of Us8A and Its Phosphorylation Mediated by Us3 in Herpes Simplex Virus 1 Pathogenesis. J Virol 2016 90: 5622-35
[Non-patent Literature 54]
   Polcicova, K. et al., Herpes keratitis in the absence of anterograde transport of virus from sensory ganglia to the cornea. Proc Natl Acad Sci U S A 2005 102: 11462-7
[Non-patent Literature 55]
   Jerome, KR. et al., Herpes simplex virus inhibits apoptosis through the action of two genes, Us5 and Us3. J Virol 1999 73: 8950-7
[Non-patent Literature 56]
   Peters, GA. et al., Inhibition of PACT-mediated activation of PKR by the herpes simplex virus type 1 Us11 protein. J Virol 2002 76: 11054-64
[Non-patent Literature 57]
   Schust, DJ. et al., Herpes simplex virus blocks intracellular transport of HLA-G in placentally derived human cells. J Immunol 1996 157: 3375-80
[Non-patent Literature 58]
   Galocha, B. et al., The active site of ICP47, a herpes simplex virus-encoded inhibitor of the major histocompatibility complex (MHC)-encoded peptide transporter associated with antigen processing (TAP), maps to the NH2-terminal 35 residues. J Exp Med 1997 185: 1565-72
[Non-patent Literature 59]
   Jugovic, P. et al., Inhibition of major histocompatibility complex class I antigen presentation in pig and primate cells by herpes simplex virus type 1 and 2 ICP47. J Virol 1998 72: 5076-84

### Summary of Invention

### Technical Problem

As described earlier, antiviral agents such as acyclovir are used for treatment of HSV. However, these antiviral agents cannot completely eliminate a virus and have a risk of reactivation of the virus due to, for example, a reduction in adherence. Thus, development of a prophylactic vaccine to prevent HSV infection itself or a therapeutic vaccine to alleviate relapse symptoms is desired. However, currently, there is no effective vaccine, and unmet medical needs for effective vaccines are high.

The present invention has an object to provide a recombinant live attenuated vaccine that can be used for prevention and treatment of an HSV-1 infection, that has enhanced immune inducibility of HSV-1 through mutation introduction, and that has high safety by introducing a mutation into a viral gene responsible for pathogenicity.

### Solution to Problem

On the basis of the hypothesis that a recombinant live attenuated vaccine which has high safety and more enhanced immune inducibility can be constructed by introducing a mutation into a virulence factor and an immune evasion factor of HSV-1, the inventors of the present invention produced a variety of mutated viruses and carried out animal experiments for evaluation. As a result, the inventors of the present invention found the following: In a mouse primary infection model infected with genital herpes, a Us3/UL13 double mutant virus obtained by further introducing a mutation (inactivation) into UL13 with respect to a virus into which a mutation (inactivation) is introduced into a gene Us3 of herpes simplex virus type 1 exhibits a superior primary infection prevention effect (virus shedding suppression effect or disease prevention effect) as compared with a traditional vaccine prototype live attenuated virus ΔTk (thymidine kinase-deficient)-inoculated group and a Us3 single mutant live attenuated virus-inoculated group which have the same origin. Note that ΔTk is focused as a comparison target because Non-patent Literatures 23 to 25 suggest safety and effectiveness of ΔTk.

Thus, the inventors of the present invention completed the present invention by finding that it is possible obtain a recombinant live attenuated vaccine which has enhanced immune inducibility by introducing a mutation (inactivation) into two or more types of genes of herpes simplex virus type 1 and which has enhanced safety by attenuating pathogenicity.

That is, a virus in accordance with an aspect of the present invention is a multiple mutant virus in which: two or more genes of herpes simplex virus type 1 (HSV-1) are modified; and a modification of the two or more genes involves a loss or reduction in gene function.

### Advantageous Effects of Invention

In accordance with an aspect of the present invention, in a case where immunity is induced with recombinant live attenuated herpes simplex virus type 1 and a vaccine containing the recombinant live attenuated herpes simplex virus type 1, a superior primary infection prevention effect can be exhibited as compared with a case where immunity is induced with a traditional live attenuated virus ΔTk which has the same origin as that of the recombinant live attenuated herpes simplex virus type 1. Thus, a high prevention effect on an HSV infection can be expected.

### Brief Description of Drawings

Fig. 1 is a schematic view of a mouse primary infection prevention test in Example 4.
Fig. 2 is a diagram showing a virus shedding suppression effect of the mouse primary infection prevention test in Example 4.
Fig. 3 is a diagram showing a pathological score in the mouse primary infection prevention test in Example 4.
Fig. 4 is a schematic view of a mouse intracerebral infection test in Example 5.
Fig. 5 is a diagram showing a survival rate in the mouse intracerebral infection test in Example 5.
Fig. 6 is a schematic view of a mouse nasal infection test in Example 6.
Fig. 7 is a diagram showing a survival rate in the mouse nasal infection test in Example 6.

### Description of Embodiments

In the present specification, "A to B" means not less than A and not more than B unless otherwise specified.

### [Multiple mutant virus]

A multiple mutant virus in accordance with an aspect of the present invention (hereinafter sometimes referred to as "recombinant live attenuated herpes simplex virus type 1") is a virus in which two or more genes of herpes simplex virus type 1 (HSV-1) are modified.

In the present specification, a modification of a gene refers to a loss or reduction in gene function. Examples of a loss or reduction in gene function include: a loss or reduction caused by the absence of expression of a protein encoded by a target gene, due to a gene deletion, a start codon substitution, or introduction of a stop codon directly below the start codon; and a loss or reduction in gene function caused by an amino acid substitution due to a change in base sequence of a gene (e.g., a deletion, substitution, or insertion of one to several tens of bases in a coding region).

HSV-1 may be a laboratory strain or a clinical isolate.

Recombinant live attenuated herpes simplex virus type 1 is preferably configured such that a gene related to immune evasion or pathogenicity expression is modified in terms of high immune inducibility and high safety. Examples of the gene related to immune evasion or pathogenicity expression include RL1 (Non-patent Literatures 26 and 27), RL2 (Non-patent Literatures 28 and 29), UL2 (Non-patent literature 30), UL3, UL4 (Non-patent literature 31), UL12 (Non-patent literature 32), UL12.5, UL13 (Non-patent literature 21), UL24 (Non-patent literature 33), UL27 (Non-patent Literatures 17 and 34 of a point mutant), UL35 (Non-patent literature 35 of a deletion variant and a point mutant), UL41 (Non-patent literature 36 of a deletion variant, Non-patent literature 37 reviewing a function, Non-patent Literature 38 of a point mutant), UL43, UL44 (Non-patent Literatures 39 and 40), UL45 (Non-patent literature 41), UL46 (Non-patent Literatures 42 and 43), UL50 (Non-patent literature 44 reporting a function and Non-patent literature 45 of a point mutant), UL56 (Non-patent Literatures 46 and 47), Us2 (Non-patent literature 48), Us3 (Non-patent literature 15 of a deletion variant and Non-patent literatures 16 to 20 of a point mutant), Us4 (Non-patent Literatures 49 and 50), Us5 (Non-patent Literatures 51 and 52), Us8A (Non-patent literature 53), Us9 (Non-patent literature 54), Us11 (Non-patent Literatures 55 and 56), and Us12 (Non-patent Literatures 57 to 59). At least one gene of Us3 and UL13 is preferably modified, and at least Us3 and UL13 are more preferably modified.

Us3 is a gene encoding a protein kinase. Examples of an amino acid sequence of a protein encoded by Us3 include an amino acid sequence of SEQ ID NO: 1 (NCBI Reference Sequence: GU 734771.1).

UL13 is a gene encoding a protein kinase. Examples of an amino acid sequence of a protein encoded by UL13 include an amino acid sequence of SEQ ID NO: 2 (NCBI Reference Sequence: GU 734771.1).

In terms of high immune inducibility and high safety, a modification of Us3 is preferably a modification of a lysine residue (K220) at position 220 in the amino acid sequence of SEQ ID NO: 1. A modification of UL13 is preferably a modification of a lysine residue (K176) at position 176 in the amino acid sequence of SEQ ID NO: 2. The recombinant live attenuated herpes simplex virus type 1 is more preferably configured such that K220 in SEQ ID NO: 1 and K176 in SEQ ID NO: 2 are modified.

In the present specification, a modification of an amino acid residue refers to a deletion or substitution of an amino acid residue. Examples of the modification of K220 in SEQ ID NO: 1 include a substitution of a lysine residue with a methionine residue. Examples of the modification of K176 in SEQ ID NO: 2 include a substitution of a lysine residue with a methionine residue.

The recombinant live attenuated herpes simplex virus type 1 can be obtained by a known genetic modification method such as a genetic modification method carried out by using homologous recombination in cultured cells, a genetic modification method using a cosmid, or a genetic modification method using a bacterial artificial chromosome (BAC) system.

### [HSV-1 vaccine]

An HSV-1 vaccine in accordance with an aspect of the present invention is a vaccine containing the recombinant live attenuated herpes simplex virus type 1 as an antigen. The HSV-1 vaccine can be used for treatment or prevention of an HSV-1 infection. In the present specification, "treatment or prevention of an HSV-1 infection" includes, for example, alleviation or prevention of aggravation of one or more symptoms related to HSV-1 infection, a reduction in onset of symptoms after HSV-1 infection, prevention, delay, or stopping of infection of cells with HSV-1 in vivo, and a reduction in number of HSV-1 in vivo.

Examples of a symptom related to HSV-1 infection include: herpes labialis; corneal herpes; genital herpes; neonatal systemic herpes; stomatitis, skin disease, encephalitis, meningitis, and myelitis each caused by HSV-1; and neurodegenerative diseases that may be caused by HSV-1.

The HSV-1 vaccine in accordance with an aspect of the present invention is superior in safety or effectiveness to a vaccine containing, as an antigen, HSV-1 in which only Us3 is modified.

The HSV-1 vaccine in accordance with an aspect of the present invention may contain one type or two or more types of the recombinant live attenuated herpes simplex virus type 1.

The HSV-1 vaccine in accordance with an aspect of the present invention may contain a pharmaceutically acceptable carrier in accordance with a purpose, use, administration route, and/or the like. The carrier can be a carrier that is normally used in production of a vaccine. Examples of the carrier include an adjuvant, a solvent, a thickener, a binder, a colorant, a stabilizer, a pH adjuster, a diluent, an excipient, a buffer, a tonicity agent, a soothing agent, a preservative, and an antioxidant.

Examples of a dosage form of the HSV-1 vaccine in accordance with an aspect of the present invention include powder formulations such as a freeze-dried formulation and a vacuum-dried formulation, liquid formulations, a capsule, an injection, an aerosolized agent, a spraying agent, a suppository, and a nasal spray. In terms of performance as a vaccine dosage form and a mechanism by which an effect is exhibited, a preferable dosage form of the HSV-1 vaccine in accordance with an aspect of the present invention is an injection, an aerosolized agent, or a nasal spray.

The content of the recombinant live attenuated herpes simplex virus type 1 contained in the HSV-1 vaccine in accordance with an aspect of the present invention can be determined, as appropriate, by, for example, a type, age, body weight, a state, and a duration of treatment of an administration subject.

A dose of the recombinant live attenuated herpes simplex virus type 1 contained in the HSV-1 vaccine in accordance with an aspect of the present invention is preferably 10³ pfu to 10⁸ pfu, more preferably 10⁴ pfu to 10⁷ pfu, and even more preferably 10⁵ pfu to 10⁶ pfu, in terms of safety and effectiveness.

### (HSV-1 vaccine administration method/ administration route)

An administration method (administration route) for the HSV-1 vaccine in accordance with an aspect of the present invention can be determined, as appropriate, by, for example, age, a state, and a duration of treatment of an administration subject. Specifically, the administration route may be either oral administration or parenteral administration, but is preferably parenteral administration. Examples of a route of parenteral administration include intravenous administration, subcutaneous administration, intradermal administration, intramuscular administration, nasal administration, dermal administration, rectal administration, respiratory tract administration, vaginal administration, and ophthalmic administration. In terms of performance as a vaccine dosage form and a mechanism by which an effect is exhibited, a preferable administration route for the HSV-1 vaccine in accordance with an aspect of the present invention is nasal administration. A vaccine for nasal administration is administered via the nasal mucosa by, for example, spraying, application, or instillation of a vaccine into the nasal mucosa.

A subject of administration of the HSV-1 vaccine in accordance with an aspect of the present invention is a subject that is infected with an HSV-1 virus or that is potentially infected with an HSV-1 virus. Examples of the subject of administration of the HSV-1 vaccine include mammals, birds, reptiles, and amphibians, and among these, mammals are preferable. Examples of the mammals include humans and non-human animals. Examples of the non-human animals include domestic animals such as cattle, horses, pigs, and sheep, and pet animals or laboratory animals such as dogs, cats, rats, mice, hamsters, monkeys, and rabbits. Preferable examples of the subject of administration of the HSV-1 vaccine include humans. Examples of the birds include poultry such as chickens, wild ducks, and domestic ducks.

The number of times of administration and a timing of administration of the HSV-1 vaccine in accordance with an aspect of the present invention can be determined, as appropriate, by, for example, a type, age, body weight, and a state of an administration subject.

A method for treating or preventing an HSV-1 infection, including a step of administering the HSV-1 vaccine in accordance with an aspect of the present invention to a subject is also encompassed in an aspect of the present invention.

No vaccine that has both safety and effectiveness has been developed to date, and past knowledge suggests importance of a live attenuated vaccine that makes it possible to strongly induce humoral immunity and cellular immunity. The present invention is innovative in that using a gene recombination technology has made it possible to develop a live attenuated vaccine which has high safety and high effectiveness.

### [Use as vaccine vector]

Recombinant live attenuated herpes simplex virus type 1 in accordance with an aspect of the present invention can be used also as a vaccine vector against another pathogen other than HSV-1. Examples of the another pathogen include an influenza virus and severe acute respiratory syndrome coronavirus 2 (SARS-CoV-2).

Aspects of the present invention can also be expressed as follows:
A virus in accordance with Aspect 1 of the present invention is a multiple mutant virus in which: two or more genes of herpes simplex virus type 1 (HSV-1) are modified; and a modification of the two or more genes involves a loss or reduction in gene function.

In Aspect 2 of the present invention, a virus may be configured that, in Aspect 1 of the present invention, the modification of the two or more genes is a modification of two or more genes selected from genes related to immunogenicity or immune evasion.

In Aspect 3 of the present invention, a virus may be configured that, in Aspect 2 of the present invention, the genes related to immunogenicity or immune evasion include Us3 and UL13.

In Aspect 4 of the present invention, a virus may be configured that, in any one of Aspects 1 to 3 of the present invention, a lysine residue (K220) at position 220 in an amino acid sequence of SEQ ID NO: 1 is modified, and a lysine residue (K176) at position 176 in an amino acid sequence of SEQ ID NO: 2 is modified.

A vaccine in accordance with Aspect 5 of the present invention is a herpes simplex virus type 1 (HSV-1) vaccine containing, as an antigen, a virus of any one of Aspects 1 to 4 of the present invention.

In Aspect 6 of the present invention, a vaccine is configured such that, in Aspect 5 of the present invention, the vaccine is superior in safety or effectiveness to a vaccine containing, as an antigen, HSV-1 in which only Us3 is modified.

In Aspect 7 of the present invention, a vaccine may be configured that, in Aspect 5 or 6 of the present invention, the vaccine is used for nasal administration.

In Aspect 8 of the present invention, a vaccine may be configured such that, in any one of Aspects 5 to 7 of the present invention, a dose of the virus is 10⁵ pfu to 10⁶ pfu.

Use in accordance with Aspect 9 of the present invention is use of a virus of any one of Aspects 1 to 4 of the present invention as a vaccine vector against a pathogen other than herpes simplex virus type 1 (HSV-1).

In Aspect 10 of the present invention, use may be configured such that, in Aspect 9 of the present invention, the pathogen is an influenza virus or SARS-CoV-2.

The following description will more specifically discuss an embodiment of the present invention with reference to Examples. It is a matter of course that the present invention is not limited to the Examples below and that details of the present invention can have various aspects. Further, the present invention is not limited to the embodiments, but can be altered by a skilled person in the art within the scope of the claims. An embodiment derived from a proper combination of technical means disclosed in respective different embodiments is also encompassed in the technical scope of the present invention. Furthermore, all the documents listed in the present specification are incorporated herein by reference.

### Examples

### [Example 1]

### Gene recombination in E. coli

A recombinant cassette having a kanamycin resistance gene was introduced, by an electroporation method, into HSV-BACmid-carrying E. coli (carrying a chloramphenicol resistance gene) including a gene sequence of strain F, which is an HSV-1 wild-type virus. In 1 mL of an LB medium, shaking culture was carried out at 200 rpm at 32°C for 30 minutes, and bacteria were applied to a chloramphenicol and kanamycin-containing LB agar medium and were cultured overnight at 32°C.

Grown clones were used to carry out PCR, and a clone with a desired sequence introduced therein was selected. The selected clone was subjected to shaking culture overnight at 32°C and 200 rpm in 1 mL of an LB medium. 100 µL of a culture solution was added to 2 mL of a chloramphenicol-containing LB medium to carry out shaking culture at 32°C and 200 rpm for 2.5 hours. 400 µL of 10% arabinose was added to carry out shaking culture at 32°C and 200 rpm for 1 hour. Shaking culture was carried out in a warm bath at 42°C and 50 rpm for 30 minutes, and shaking culture was carried out at 32°C and 200 rpm for 1.5 hours. 10 µL of the culture solution was diluted in 1 mL of ultrapure water, and 10 µL of a diluent was diluted in 1 mL of ultrapure water. After that, 150 µL of a diluent was applied to a chloramphenicol-containing LB agar medium and cultured overnight at 32°C. The grown clones were streaked into the chloramphenicol-containing LB agar medium and the chloramphenicol and kanamycin-containing LB agar medium and cultured overnight at 32°C. A clone growing only in the chloramphenicol-containing LB medium was selected by PCR.

### [Example 2]

### Purification of BACmid

A selected clone was inoculated into 200 mL of a chloramphenicol-containing LB medium and cultured overnight at 32°C and 200 rpm. Centrifugation was carried out at 4°C and 7,000 rpm for 5 minutes to remove a supernatant. A pellet was suspended in 5 mL of Sol I (0.9% D-glucose, 25 mM Tris-HCl (pH 8.0), and 10 mM EDTA), and 10 mL of Sol II (0.1% SDS and 0.2 N NaOH) was added. After inversion mixing, incubation was carried out at room temperature for 10 minutes. 7.5 mL of Sol III (29.43% potassium acetate and 11.5% glacial acetic acid) was added to carry out inversion mixing. Centrifugation was carried out at 4°C and 15,000 rpm for 20 minutes to filter a supernatant. A 0.6-fold amount of 2-propanol was added to carry out inversion mixing. Centrifugation was carried out at 4°C and 5,000 rpm for 5 minutes to remove a supernatant. A pellet was suspended in 4 mL of Tris-EDTA (TE), and 5.4 g of cesium chloride was added. 180 µL of a 10 mg/mL ethidium bromide solution was added to carry out ultracentrifugation overnight at 20°C and 70,000 rpm.

Among bands produced by ultracentrifugation, a desired band was harvested with a 1-mL syringe with an 18-G needle, and an equal amount of 99.5% ethanol was added. After inversion mixing, centrifugation was carried out at 4°C and 15,000 rpm for 2 minutes to remove a supernatant. A pellet was suspended in 400 µL of TE, and 500 µL of a 1:1 mixed solution of phenol and chloroform was added. After inversion mixing, centrifugation was carried out at 4°C and 15,000 rpm for 3 minutes. An aqueous layer was transferred to a new tube to carry out a similar process, and the aqueous layer was transferred to a new tube. An equal amount of diethyl ether was added to carry out inversion mixing. After that, centrifugation was carried out at 4°C and 15,000 rpm for 3 minutes. An ether layer was removed, and a similar process was carried out three times. The ether layer was removed, and 40 µL of 3M sodium acetate (pH 5.2) and 1 mL of 99.5% ethanol were added to carry out inversion mixing. Centrifugation was carried out at 4°C and 15,000 rpm for 10 minutes to remove a supernatant. 1 mL of 70% ethanol was added to carry out inversion mixing. After that, centrifugation was carried out at 4°C and 15,000 rpm for 2 minutes to remove a supernatant. Centrifugation was carried out again under the same condition to remove a supernatant.

### [Example 3]

### Virus reconstruction in rabbit skin cell

Suspension in 250 µL sterile ultrapure water was carried out, and 35 µL of a 2.2 M calcium chloride solution was added to carry out mixing by tapping. 250 µL of 2× HBS (1.6% sodium chloride, 0.074% potassium chloride, 0.027% sodium hydrogen phosphate dihydrate, 0.2% dextrose, and 1% HEPES (pH 7.05)) was added to carry out mixing by tapping (a DNA solution). Ten minutes later, a medium was removed from rabbit skin cells cultured in a T-25 flask, and washing was carried out with 5 mL of PBS. 5 mL of 0.04% DEAE-dextran was added to carry out incubation at 37°C under 5% CO₂ for 10 minutes. The DEAE-dextran was removed, and 5 mL of TEN (0.2 M sodium chloride, 20 mM Tris-HCl (pH8.0), and 2 mM EDTA) was added and removed. 5 mL of a 5% FCS-containing DMEM medium was added, and the DNA solution was added. Four hours later, the medium was removed, and 5 mL of the 5% FCS-containing DMEM medium was added and removed. 5 mL of 5% FCS-containing DMEM was added to carry out incubation overnight at 37°C under 5% CO₂. The medium was removed, and 5 mL of a 199 V medium was added to carry out incubation at 37°C under 5% CO₂.

As an indicator of virus production, it was observed whether there was any plaque. Upon successful confirmation of virus production, Vero cells were infected with a virus at an appropriate timing and subjected to expanded culture. A viral titer was measured by a plaque assay. A "recombinant live attenuated vaccine" (hereinafter referred to as an "HSV-1 vaccine") containing a resulting virus as an antigen was produced and used for an experiment.

### [Example 4]

### Mouse primary infection prevention test

Mice were nasally inoculated with 1×10³ pfu of an HSV-1 vaccine. Three weeks later, 8.35 mg/mL of Depo-Gestin was subcutaneously administered. One week later, 1×10⁷ pfu of HSV-1 wild-type strain F was vaginally challenged. A vaginal lesion score was evaluated for 2 weeks, and a vaginal douche was collected to measure a viral titer by a plaque assay. The vaginal lesion score was evaluated as follows: 0: no symptom; 1: slight genital erythema and/or edema; 2: genital alopecia, erythema, and/or edema; 3: marked genital alopecia, erythema, and/or edema; 4: hind-limb paralysis; and 5: death.

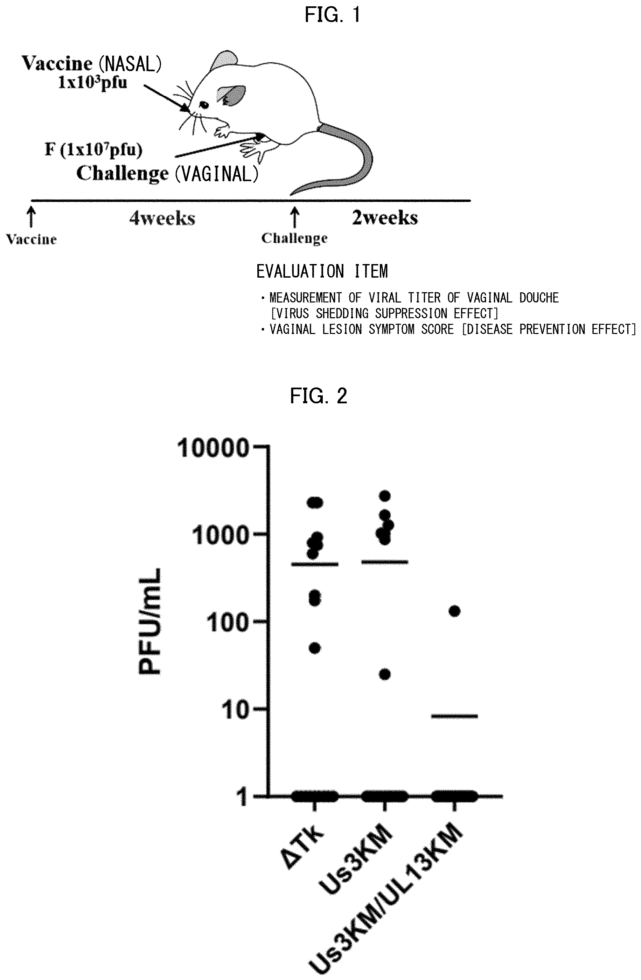

Fig. 1 shows a schematic view of a mouse primary infection prevention test. The HSV-1 vaccine containing Us3KM/UL13KM as an antigen further suppressed virus shedding than a traditional vaccine prototype live attenuated virus ΔTk-inoculated group (Fig. 2). Note here that "Us3KM" means a virus in which the 220th lysine in Us3 is converted to methionine, that "UL13KM" means a virus in which the 176th lysine in UL13 is converted to methionine, and that "Us3KM/UL13KM" means a double mutant virus of Us3KM and UL13KM.

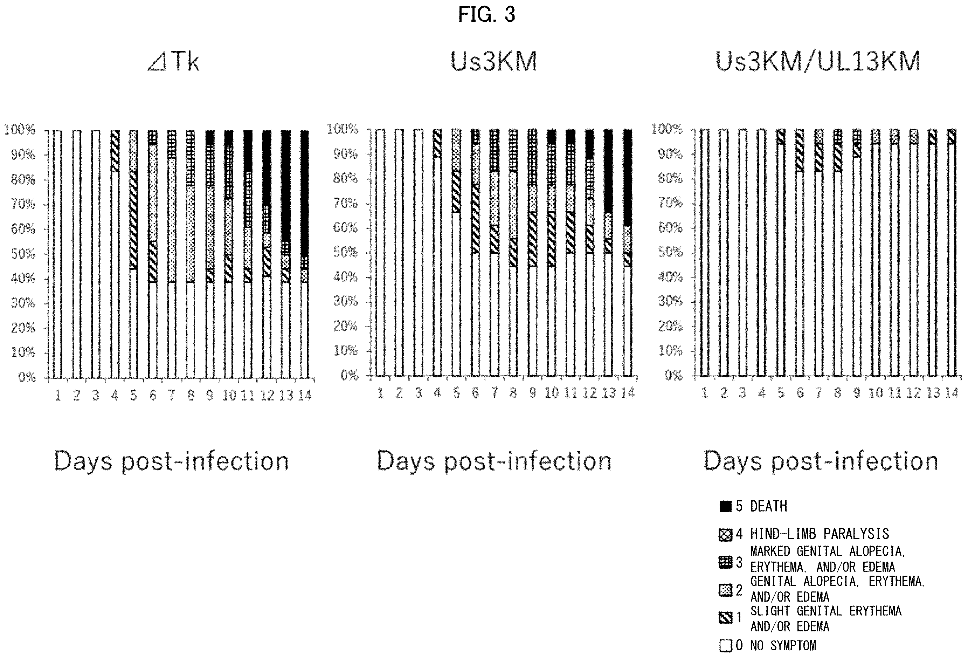

A Us3KM/UL13KM-inoculated group also had significantly reduced symptoms of a vaginal lesion as compared with the ΔTk-inoculated group and exhibited a favorable onset prevention effect (Fig. 3).

### [Example 5]

### Mouse intracerebral infection test

Mice were intracerebrally inoculated with 1×10⁵ pfu of an HSV-1 vaccine. A survival rate was observed for 2 weeks.

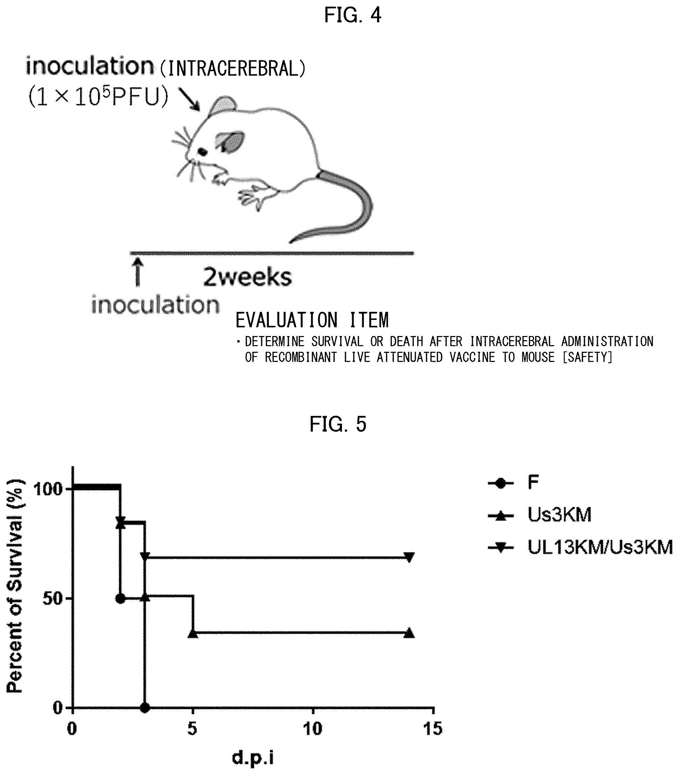

Fig. 4 shows a schematic view of a mouse intracerebral infection test. A survival rate after administration of the HSV-1 vaccine containing Us3KM/UL13KM as an antigen was higher than that in wild-type virus strain F- and Us3KM single mutant virus-inoculated groups, and attenuation of pathogenicity was determined (Fig. 5).

### [Example 6]

### Mouse nasal infection test

Mice were nasally inoculated with 1×10⁶ pfu of an HSV-1 vaccine. A survival rate was observed for 2 weeks.

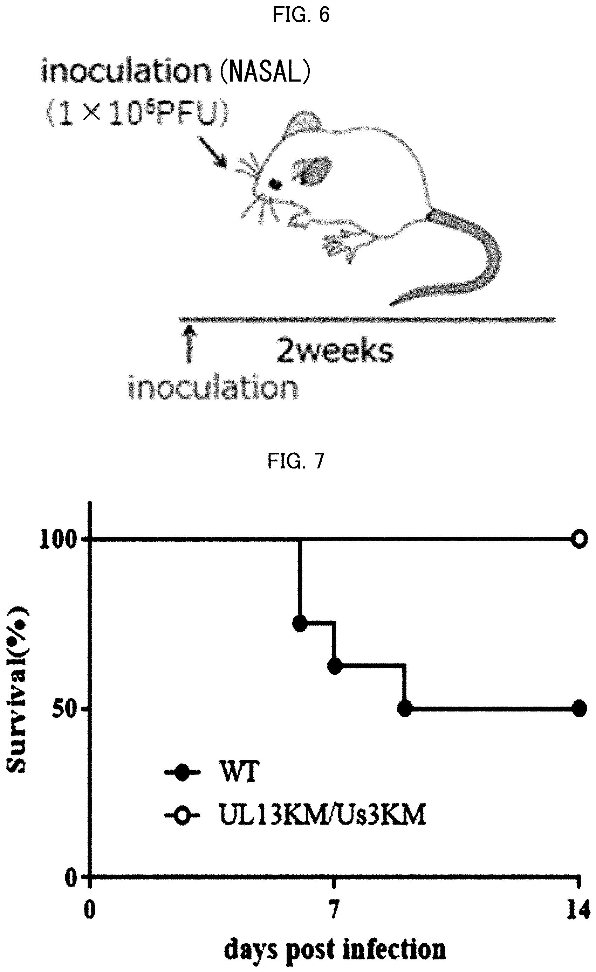

Fig. 6 shows a schematic view of a mouse nasal infection test. A survival rate after administration of the HSV-1 vaccine containing Us3KM/UL13KM as an antigen was higher than that in a wild-type virus strain F-inoculated group, and attenuation of pathogenicity was determined (Fig. 7).

### Industrial Applicability

An HSV-1 vaccine of the present invention obtained by introducing a mutation into two types of genes Us3 and UL13 of herpes simplex virus type 1 is expected to be industrially used as a vaccine that has both high safety and high effectiveness.

## Claims

1. A multiple mutant virus wherein:
two or more genes of herpes simplex virus type 1 (HSV-1) are modified; and
a modification of the two or more genes involves a loss or reduction in gene function.

2. The virus as set forth in claim 1, wherein the modification of the two or more genes is a modification of two or more genes selected from genes related to immunogenicity or immune evasion.

3. The virus as set forth in claim 2, wherein the genes related to immunogenicity or immune evasion include Us3 and UL13.

4. The virus as set forth in claim 3, wherein a lysine residue (K220) at position 220 in an amino acid sequence of SEQ ID NO: 1 is modified, and
a lysine residue (K176) at position 176 in an amino acid sequence of SEQ ID NO: 2 is modified.

5. A vaccine against herpes simplex virus type 1 (HSV-1), comprising, as an antigen, a virus recited in any one of claims 1 to 4.

6. The vaccine as set forth in claim 5, wherein the vaccine is superior in safety or effectiveness to a vaccine containing, as an antigen, HSV-1 in which only Us3 is modified.

7. The vaccine as set forth in claim 5, wherein the vaccine is used for nasal administration.

8. The vaccine as set forth in claim 5, wherein a dose of the virus is 10⁵ pfu to 10⁶ pfu.

9. Use of a virus recited in any one of claims 1 to 4 as a vaccine vector against a pathogen other than herpes simplex virus type 1 (HSV-1).

10. The use as a vaccine vector as set forth in claim 9, wherein the pathogen is an influenza virus or SARS-CoV-2.
